# EUROPEAN PATENT SPECIFICATION

(11) **EP 0 446 850 B1**
(45) Date of publication and mention of the grant of the patent: **24.01.1996**
(21) Application number: 91103719.0
(22) Date of filing: 12.03.1991
(51) Int. Cl.: C07K 1/00

(54) **Process for purifying recombinant human beta-interferon**
Verfahren zur Herstellung von rekombinantem Humaninterferon-beta in reiner Form
Procédé de purification de l'interféron bêta humain recombinant

(30) Priority: 16.03.1990 IT 1969990
(43) Date of publication of application: 18.09.1991
(73) Proprietor: SCLAVO S.p.A., I-53100 Siena (IT)
(72) Inventor: Protasi, Otello, I-53100 Siena (IT); Rappuoli, Paolo, I-53034 Colle val D'Elsa,(Siena) (IT)
(74) Representative: Gervasi, Gemma, Dr.

(56) References cited:
- EP-A- 0 117 470
- EP-A- 0 301 314
- WO-A-88/04937
- DNA, vol. 3, no. 4, 1984, pages 297-308, New York, US; Y. CHERNAJOVSKY et al.:"Efficient constitutive production of human fibroblast interferon by hamstercells transformed with the IFN-beta gene fused to an SV40 early promoter"

## Description

The present invention relates to a purification process for recombinant human β-interferon (rhu-β-IFN) by the combined use of chromatography on siliceous matrix and cation exchange chromatography.

The interferons, briefly designated IFN, are glycoprotein substances which are produced by different human and animal cells as a response to viral and non viral agents during the first phases of an infection. The interferons develop different biological activities as, for instance, an antiviral, antiproliferative, anticellular and immunomodulator activity.

On the basis of their biological activities at least three types of interferon have been identified and characterized: namely alfa, beta and gamma (hereinafter designated as α, β and γ), which are produced by leucocytes, fibroblasts, lymphocytes and by immunosystem. In particular, the human β-interferon (hu-β-IFN) is produced and, secreted by the fibroblasts, and it is applied as a coadjuvant of secondary immunodeficiencies in viral infections and in the oncological domain.

Methods for the production of β-interferon by the in vitro culture of human fibroblasts in conditions of superinduction with poly-IC (polyribosinic acid and polyribocytidylic acid) and following purification by chromatographic and electrophoretic techniques are known in the prior art. Said methods, however, show drawbacks due to the low production yield and, specifically, to the poor availability of the starting material (fibroblasts). The current developments in the technical field of genetic engineering have enabled the high yield production of proteins and polypeptides by fermentation processes making use of transformed host organisms harbouring a vector comprising the gene encoding the product of interest.

Many processes for the production of recombinant human β-interferon based on the use of different manipulated host organisms have been described in the technical and patent literature (see Derynck, R. et al. (1980), Nature 285, 542-547; Derynck, R. et al. (1980), Nature 287, 193-197; Taniguchi, T. et al. (1980), Gene 10, 11-15; Taniguchi, T. et al. (1980), PNAS 77, 4003-4006; EP-A-041313). Said known processes bring about many problems relating to the purification of the obtained recombinant products. It is in fact known that the application of proteins in human therapy requires the availability of products with high levels of purity and specific activity, free from toxic cellular contaminants and/or foreign chemical substances used in the step of extraction and purification of the product. Purification methods like precipitation, electrophoresis, affinity chromatography or ionic exchange chromatography are well known in the technical prior art and have been applied for purifying proteins from cellular extracts and secrets. In particular many purification processes for natural and recombinant β-interferon have been described in the technical and patent literature. In this connection applications EP-A-063482, GB-A-1 534 812 and the paper by Billiau et al., Antimicrob. Agents and Chemoterapy, 16, 49-55, (1979) are referred to. Specifically the EP-A-063482 discloses a method for the purification of β-interferon comprising the use in succession of the following chromatographic techniques:
1) CPG, 2) Concanavalin-A-Sepharose^{(R)}, 3) Heparin-Sepharose^{(R)} or Procion Red-A-Agarose and 4) gel filtration. The GB-A-1 534 812 discloses a process for the purification of β-interferon comprising as the main feature the use of CPG and of a strongly acid eluent (pH 2.0). According to this process a 40 to 90-fold purification of the crude β-interferon and a yield of 60% is obtained. The process according to Billiau et al. (supra) allows to obtain interferon in a total yield of 50% and with a specific activity of 1x10⁶ Units/mg protein.

In conclusion said earlier processes are not completely satisfactory because of the low production and purification yields and because of the many necessary steps.

Therefore these processes are not suitable for an industrial scale application.

It has now been discovered that the drawbacks of the prior art methods can be overcome by a purification process for recombinant human β-interferon, which is simple and economically advantageous. Thus an object of the present invention is a purification process for the recombinant human β-interferon from solutions comprising the same in unrefined state by combining chromatography on siliceous matrix and cation exchange chromatography.

A further object of the present invention is the use of the recombinant human β-interferon purified according thereto as active agent for manufacturing pharmaceutical compositions for the treatment of viral infections and tumors and as immunomodulator.

A still further object of the present invention is a pharmaceutic composition comprising a therapeutically effective amount of recombinant human β-interferon purified according to the present process.

Other objects of the present invention will be evident in the light of the description and examples which follow.

In details the process of chromatographic purification according to the present invention consists in that:
a) an aqueous solution of recombinant human β-interferon in unrefined state is contacted with a siliceous solid matrix equilibrated with deionized water whereon the recombinant human β-interferon is adsorbed; said interferon is then eluted by using an aqueous solution of acetic acid at molar concentration comprised between 2 and 10 mM/l and the eluted fractions containing the recombinant human β-interferon of higher purity level are then collected; and
b) the eluted fractions containing the recombinant human β-interferon purified according to stage a) are loaded with a flow rate not exceeding 0.1 cm/min on a cation exchange resin equilibrated with acetate buffer at a pH not lower than 3.0; thereafter the rhu-β-interferon adsorbed upon said matrix is eluted at a flow rate not higher than 0.1 cm/min with phosphate buffer of concentration comprised between 0.07 and 0.15 moles/litre, at a pH comprised between 6.2 and 6.8 and containing 0.15 to 0.18 moles/litre of NaCl. Recombinant human β-interferon is obtained in this way as a unique clear peak in determined eluted fractions.

Insofar as the recombinant human β-interferon to be purified is concerned, this may be obtained by culturing host organisms selected from the group of bacteria, yeasts or mammal cells transformed with an expression vector comprising the gene coding for human β-interferon and by separating the recombinant product from the cell lysate or from the culture medium.

According to an embodiment of the process of the present invention, rhu-β-IFN is produced by CHO cells (chinese hamster ovary tumor cells, clone CHO β-IFNg 454 derived from the transfection of the line CHO DXB11 with the plasmid pSAD β-IfNg1 from CHIRON) on microcarriers in a bioreactor. The transformed cells express and secret into the culture medium the recombinant human β-interferon in impure state having a specific activity of about 10⁴ U.I./mg protein. The optimum sizes (section diameter and length) of the chromatographic column, for the clear separation of the constituents of the rhu-β-IFN solution depend, once defined the operative conditions for the flow rate and the bond ratio between the recombinant human β-interferon and the solid support, on the amount of the product intended to be purified.

### Stage a).

In stage a) of the process of the present invention the siliceous materials are selected among those capable of binding the recombinant human β-interferon and are commercially available. Preferred materials for the purpose of the present invention are controlled porosity glass materials (CPG) and among those, particularly preferred is the CPG 500 from Electro Nucleonics. The amount of siliceous material, and in particular of CPG, used to prepare the column is determined in such a way as to have a bond ratio U.I. of loaded β-interferon per ml adsorbent material as high as possible in order to obtain the following advantages: - full adsorption of the loaded interferon; - sharpness of the peak corresponding to the purified recombinant human β-interferon; - obtaining of fractions containing high title of pure β-IFN. Once the column is prepared, it is sterilized by statically contacting the same with a 10% (w/v) aqueous formaldehyde solution for about 4 hours. Thereafter the column is washed with deionized sterile pyrogen-free water until complete removal of the formaldehyde, determined according to the method of Critchfield, F.E. et al. (1957), Anal. Chem. 29, 797, and it is cooled at 4°C.

The solution of crude recombinant hu-β-IFN is then fed at the linear rate (flow/volume of adsorbent material) of 1 to 2 cm/min in order to obtain the adsorption of the crude interferon upon the siliceous material. The alimentation is carried out at atmospheric pressure and at a temperature of, or about, 4°C. During the loading phase the interferon is bound to the siliceous material, whereas most of the foreign proteins remain in solution and percolates out of the column. The pH value during the contact phase between rhu-β-IFN and the siliceous material is that of the loaded solution. At the end of the alimentation phase, the column is washed with deionized sterile pyrogen-free water until elimination of any adsorbance at 280 nm, and the remaining foreign proteins are removed by subsequently eluting the column with aqueous 1.4 M NaCl solution and with deionized sterile pyrogen-free water. The analysis of the single eluted fractions, carried out by determining the IFN biological activity according to Armstrong J.A. (1981), Methods Enzymol. 78, 381-387, does not reveal the presence of interferon, confirming in this way the stability of the link between the loaded interferon and the adsorbent material.

The use of deionized water to equilibrate and to wash the column turned out to be particularly advantageous for the purpose of the purification process of the present invention. It was in fact realized that the buffer solutions, generally used in the prior art, cause the interferon elution to be delayed when eluting acid solutions at low molar concentration are used.

This is likely due to the fact that the column remains buffered and therefore the pH values necessary to release the rhu-β-IFN from the adsorbent material with an eluting solution having rather low molarity are achieved more slowly.

The rhu-β-interferon is thereafter released from the adsorbent material by eluting with an aqueous solution of acetic acid at a molar concentration comprised between 2 and 10 mM/l, preferably between 4 and 7 mM/l. An acetic acid concentration of 5 mM/l is particularly preferred since it allows the rhu-β-IFN to be obtained in, or about, 80% yield, considering fractions with an activity higher than 1x10⁷ U.I./mg. The low acetic acid molarity applied to elute the rhu-β-IFN is particularly favourable for the development of the successive cation exchange chromatography. The fractions containing the partially purified β-interferon coming from step a) show in fact pH values ranging from 3.5 to 4.0 which is sufficiently low to guarantee that the interferon is bound to the employed cationic exchange matrix, but at the same time, not so low to raise problems of ineffectiveness of the matrix due to extreme acidity (pH lower than 3.0).

Operating at the preferred conditions, it was observed that by increasing the units of rhu-β-IFN bound per ml of CPG from 3.5x10⁶ to ca. 50x10⁶ U.I., the acetic acid molarity necessary to completely elute the interferon bound to CPG decreases proportionally.

At the end of the elution phase, the effluent is collected in fractions of suitable volume, and their content is analysed according to conventional techniques. If desired, the column may be regenerated by treatment with a 5 M aqueuos acetic acid solution and successive washings with deionized sterile pyrogen-free water.

### Stage b).

The cation exchange chromatography according to the process of the present invention comprises:
1)loading the eluted fractions containing the recombinant human β-interferon partially purified according to stage a) at a flow rate not exceeding 0.1 cm/min on a cation exchange matrix equilibrated with acetate buffer at a pH not lower than 3.0;
2) treating the column with the same acetate buffer comprising 0.5 M NaCl;
3) washing the column with acetate buffer at pH not lower than 3.0 until complete removal of the Cl⁻ ions; and
4) eluting with flow rate not exceeding 0.1 cm/min the recombinant human β-interferon adsorbed upon said matrix with phosphate buffer of a concentration between 0.07 and 0.15 moles/litre, at a pH between 6.2 and 6.8 and containing 0.15 to 0.18 moles/litre of NaCl.

To perform the cation exchange chromatography, resins, selected amoung CM-Sepharose^{(R)}, CM-Sephadex(R), SP-Sepharose^{(R)}, SP-sephadex^{(R)}, or CM-Accel^{(R)}, which are commercially available, may be used. Preferably CM-Sepharose-CL-6B is used.

According to the present invention the best bond ratio between rhu-β-IFN and the selected matrix was found to be 0.2 to 0.3 mg/ml. Particularly critical for the purpose of the present invention are moreover the loading and elution flow of recombinant human β-IFN. It was in fact observed that feeding at a flow rate higher than 0.1 cm/min, higher molarities of NaCl are necessary to elute the recombinant human β-IFN from the column with consequent decrease of the purity degree. Likewise, eluting at a flow rate higher than 0.1 cm/min, an extremely low yield in rhu-β-IFN with a specific activity exceeding 2x10⁸ U.I./mg is obtained. Therefore the loading and eluting flow rates should preferably not be higher than 0.1 cm/min, more preferably they are comprised between 0.05 and 0.1 cm/min. According to the present invention the acetate buffer has a pH ranging between 3.0 and 4.5, preferably between 3.5 and 4.0. The molar concentration of the eluting phosphate buffer is preferably comprised between 0.09 and 0.11 moles/litre.

The recombinant human β-interferon obtained according to the process of the present invention is isolated as single peak and gives a single narrow band in SDS-PAGE electrophoresis. The molecular weight of the purified recombinant human β-interferon, determined on polyacrylamide gel electrophoresis, turned out to be about 24.000 daltons, which corresponds to that of the natural β-interferon. Furthermore the laser scanner analysis reveals a purity minimum value of 99.58%.

The recombinant human β-interferon obtained according to the process of the present invention is thus particularly suitable for administration to humans.

The rhu-β-IFN purified according to the process of the present invention may conveniently be administered orally, parenterally or endovenously for use as an antivira, antitumoral and immunomodulator.

The compositions comprising the so purified rhu-β-IFN may be prepared, according to conventional techniques, combining the active agent with an inert excipient and optionally with stabilizers and conventional additives appropriately selected.

For oral application the rhu-β-IFN purified according to the process of the present invention may be administered in the form of tablets, capsules, drops and elixir manufactured using conventional carriers/excipients like starch, sugars, water, alcohol etc. and optionally comprising flavouring, stabilizing, preservative and lubricant agents.

For parenteral or endovenous application the most preferred carrier is sterile water for injection. Additives may be included according to the known art. The therapeutically effective daily dosage will vary depending on the patient to be treated (weight, age and conditions) and on the administering way.

Therefore the pharmaceutic compositions of the present invention comprise the purified recombinant human β-interferon in amounts suitable to guarantee the effective daily dosage.

The experimental examples which follow are intended to illustrate, not however to limit the invention.

### Example 1

### Purification of hu-β-IFN recombinant with CPG.

For this purpose, a solution of, crude hu-β-IFN (600 ml) obtained from a culture of CHO cells (chinese hamster ovary tumor cells, clone CHO β-IFNg 454 derived from the transfection of the line CHO DXB11 with the plasmid pSAD β-IfNg1 from CHIRON) on microcarriers in a bioreactor has been used. The recombinant hu-β-IFN solution has the following composition:
- hu-β-IFN title 86 840 U.I./ml
- total hu-β-IFN 52.1x10⁶ U.I.
- total proteins 1.502 mg/ml
- specific activity 5.8x10⁴ U.I./ml

A glass column of 1.6 cm diameter and 0.5 cm height (Pharmacia, model C16) is packed with 1 ml CPG^{(R)}-500 (Electro Nucleonics) previously purified with:
a)- a mixture consisting of n-propanol 40%, beta-mercaptoethanol 0.07% and urea to saturation, pH 1.5;
b)- NH₄OH 5%;
c)- CH₃COOH 0.1 M;
d)- HNO₃ 65% at boiling point.

Subsequently the column is sterilized with an aqueous solution of formaldehyde (10% w/v) at room temperature (20°-25°C) for 4 hours. Then the column is washed with deionized sterile pyrogen-free water until complete removal of the formaldehyde, determined according to the method of Critchfield, F.E. et al. (1957), Anal. Chem. 29, 797, and it is cooled at 4°C. Thereafter 600 ml supernatant containing crude human β-IFN having the aforementioned composition are fed in the column at the flow rate of 1 ml/min x cm (cm/min) obtaining a ratio β-IFN/CPG of 52x10⁶ U.I./ml CPG. At the end of the alimentation phase, the column is washed with deionized sterile pyrogen-free water until elimination of any adsorbance at 280 nm, then it is eluted with aqueous 1.4 M NaCl solution and at the end it is washed with deionized sterile pyrogen-free water until elimination of the chloride ions. The flow rate is 1 cm/min. The process is further carried out with the elution of rhu-β-IFN with an acetic acid solution 5 mM (pH 3.8) fed in at the flow rate of 0.25 cm/min. The following fractions are obtained:

| β-IFN | total volume | protein | β-IFN |
|---|---|---|---|
| U.I. | ml | mg/ml | U.I./mg protein |
| 3 757 000 | 3.2 | 0.12 | 3.1×10⁷ |
| 2 004 000 | 7.2 | 0.063 | 3.2×10⁷ |
| 317 300 | 12.0 | 0.025 | 1.3×10⁷ |
| 176 000 | 16.0 | 0.013 | 1.3×10⁷ |
| 85 500 | 12.0 | n.d. | n.d. |
| 50 100 | 13.5 | n.d. | n.d. |
| 50 100 | 12.0 | n.d. | n.d. |

The total yield in recombinant human β-IFN partially purified is 85%; n.d. means not determined.

### Example 2

### Purification of recombinant human β-IFN on an industrial scale by means of CPG^{(R)}-500

The purification is carried out as described above in example 1 making use of a column 25.2 cm x 2.4 cm (Pharmacia, model BP 252) containing 1.2 1 CPG^{(R)}-500. 486 litres solution of crude recombinant hu-β-IFN having the following features:
- hu-β-IFN title 100 600 U.I./mg
- total hu-β-IFN 39.6 x 10⁹ U.I.
- proteins 1.4 mg/ml
- specific activity 5.8 x 10⁴ U.I.
   are then loaded.

The elution is effected with a 5 mM acetic acid solution and the following fractions are obtained:

| Title | protein | specific | total |
|---|---|---|---|
| β-IFN | | activity | volume |
| U.I./ml | mg/ml | U.I./mg | ml |
| 861 000 | 0.065 | 1.3×10⁷ | 400 |
| 4 223 000 | 0.177 | 2.4×10⁷ | 200 |
| 12 100 000 | 0.478 | 2.5×10⁷ | 1000 |
| 6 560 000 | 0.302 | 2.2×10⁷ | 2500 |
| 1 558 000 | 0.102 | 1.5×10⁷ | 6000 |
| 451 000 | 0.041 | 1.1×10⁷ | 7000 |

The total yield in rhu-β-IFN partially purified is 86%.

### Example 3

### Purification by cation exchange chromatography of rhu-β-IFN partially purified on CPG^{(R)}-500.

A glass column 1.6 cm diameter and 0.5 cm height (Pharmacia, model C16) is used.

The column, the flow cell, the valves and the outlets are sterilized by contacting them with ethanol 25% for a night, while all inlets and fittings upstream the column are sterilized in autoclave. The column is then washed with 0.2 M acetate buffer (pH 4.0) until elimination of the ethyl alcohol (assayed by fotometric test), then equilibrated using the same acetate buffer at a flow rate of 0.08 cm/min and packed with 17.6 ml CM-Sepharose^{(R)} CL-6B (Pharmacia) (height of the gel in the column 8.8 cm) loaded at a flow rate of 0.178 cm/min in water. The solution of recombinant human β-IFN (56.5 ml) partially purified by chromatography on CPG^{(R)}-500 according to example 1 is then loaded; the solution has the following composition:
- hu-β-IFN title 1 115 000 U.I./ml
- protein title 0.076 mg/ml
- specific activity 1.46 x 10⁷ U.I./mg
- total hu-β-IFN 63 x 10⁶ U.I.
- total proteins 4.3 mg

The solution is fed in the column at flow rate of 0.08 cm/min and a ratio mg protein/ml gel of about 0.2 is obtained. At the end of the alimentation, the column is washed with 2 volumes 0.2 M acetate buffer (pH 4.0), eluted with 5 volumes 0.5 M NaCl in 0.2 M acetate buffer and washed once again with 0.2 M acetate buffer (pH 4.0) until elimination of the chloride ions. All the solutions are fed in at flow rate of 0.08 cm/min. Thereafter the recombinant human β-IFN adsorbed upon the column is eluted using a solution 0.15 M NaCl in 0.1 M phosphate buffer (pH 6.5) at the flow rate of 0.075 cm/min and the following fractions are collected:

| Title | proteins | specific | volume | total β-IFN |
|---|---|---|---|---|
| β-IFN | | activity | ml | U.I. |
| U.I./ml | µg/ml | U.I./mg | | |
| 111 600 | 29.2 | 3.8×10⁶ | 1.5 | 0.17×10⁶ |
| 325 500 | 37.2 | 8.7×10⁶ | 3.0 | 0.97×10⁶ |
| 423 150 | 1.8 | 2.3×10⁸ | 9.0 | 3.80×10⁶ |
| 255 750 | 1.4 | 1.8×10⁸ | 4.5 | 1.10×10⁶ |
| 190 650 | 0.4 | 4.8×10⁸ | 6.0 | 1.10×10⁶ |
| 162 750 | 2.1 | 7.7×10⁷ | 6.0 | 1.06×10⁶ |
| 176 700 | 0.7 | 2.5×10⁸ | 6.0 | 1.10×10⁶ |
| 111 600 | 0.3 | 3.7×10⁸ | 6.0 | 0.67×10⁶ |
| 74 000 | 0.3 | 2.5×10⁸ | 6.0 | 0.44×10⁶ |
| 111 600 | 0.3 | 3.7×10⁸ | 6.0 | 0.67×10⁶ |
| 376 650 | 3.2 | 1.2×10⁸ | 6.0 | 2.26×10⁶ |
| 762 600 | 1.1 | 6.9×10⁸ | 6.0 | 4.58×10⁶ |
| 251 100 | 3.0 | 8.4×10⁷ | 6.0 | 1.50×10⁶ |
| 232 500 | 0.3 | 7.7×10⁸ | 6.0 | 1.39×10⁶ |
| 139 500 | 3.9 | 3.6×10⁷ | 6.0 | 0.83×10⁶ |
| 120 900 | 3.5 | 5.0×10⁷ | 6.0 | 0.72×10⁶ |
| 111 600 | 5.5 | 2.0 ×10⁷ | 6.0 | 0.67×10⁶ |
| 93 000 | 3.9 | 2.4×10⁷ | 6.0 | 0.56×10⁶ |
| 46 500 | 2.8 | 1.7×10⁷ | 6.0 | 0.28×10⁶ |
| 37 200 | - | - | - | - |
| 27 900 | - | - | - | - |

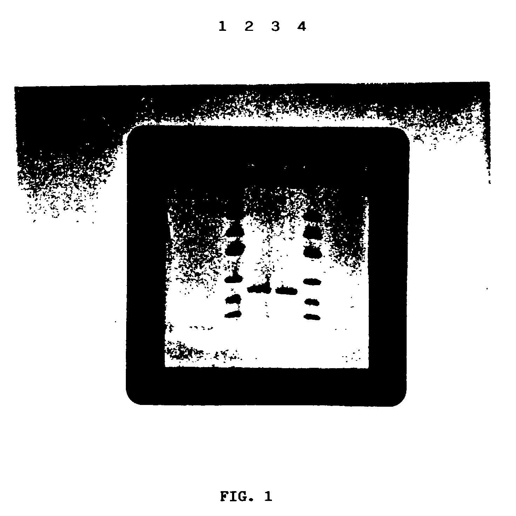

The total yield in rhu-β-IFN is about 78%, about 45% of which concerns fractions with specific activity comprised between 1x10⁸ U.I./mg and 7.7x10⁸ U.I./mg, with a final yield referred to the crude rhu-β-IFN of 38%. The protein title has been determined with BIO-RAD micro-BCA kit. The purity of specimens of recombinant human β-IFN has been assayed on 12.5% and 15% SDS polyacrylamide gel electrophoresis. After staining with Comassi Blue, one single band was obtained (Fig.1 line 2 and 3).

### Example 4

### Purification by cation exchange chromatography of rhu-β-IFN partially purified on CPG^{(R)}-500.

A column of 11.3 cm diameter and 30 cm height (Pharmacia, model BP 113/30) was packed with about 1000 ml CM Sepharose^{(R)} CL-68 (Height of the gel in the column about 10 cm) loaded at a flow rate of 0.18 cm/min in water. Then 650 ml rhu-β-IFN solution obtained according to example 2 are fed in the column at a flow rate of 0.1 cm/min; the solution has the following features:
- β-IFN title 6 560 000 U.I./ml
- protein title 0.32 mg/ml
- specific activity 2.20 x 10⁷ U.I./mg
- total β-IFN 4.26 x 10⁹ U.I.
- total proteins 208 mg.

A protein/gel ratio of 0.21 mg/ml was obtained. The elution of β-IFN adsorbed upon the column is effected with a 0.15 M NaCl in 0.1 M phosphate buffer solution (pH 6.5) at a flow rate of 0.075 cm/min. At the end of the elution the following fractions are collected:

| Title | protein | specific | volume | total β-IFN |
|---|---|---|---|---|
| β-IFN | | activity | ml | U.I. |
| U.I./ml | µg/ml | U.I./mg | | |
| 1 904 000 | 33.3 | 5.7×10⁷ | 240 | 4.56×10⁸ |
| 5 236 000 | 116.3 | 4.5×10⁷ | 30 | 1.57×10⁸ |
| 6 528 000 | 121.6 | 5.1×10⁷ | 50 | 3.26×10⁸ |
| 5 780 000 | 100.6 | 5.7×10⁷ | 60 | 3.46×10⁸ |
| 4 216 000 | 58.5 | 7.2×10⁷ | 50 | 2.10×10⁸ |
| 3 196 000 | 44.7 | 7.1×10⁷ | 50 | 1.59×10⁸ |
| 2 992 000 | 28.9 | 1.0×10⁸ | 85 | 2.54×10⁸ |
| 2 516 000 | 16.1 | 1.6×10⁸ | 150 | 3.77×10⁸ |
| 2 312 000 | 9.7 | 2.4×10⁸ | 430 | 9.90×10⁸ |
| 816 000 | 7.7 | 1.1×10⁸ | 170 | 1.38×10⁸ |
| 476 000 | 6.2 | 7.7×10⁸ | 140 | 0.66×10⁸ |
| 340 000 | 5.1 | 6.7×10⁸ | 275 | 0.93×10⁸ |
| 340 000 | 4.8 | 7.1×10⁸ | 275 | 0.93×10⁸ |
| 272 000 | 4.3 | 6.3×10⁸ | 275 | 0.75×10⁸ |
| 335 000 | 4.5 | 7.4×10⁸ | 275 | 0.92×10⁸ |
| 176 800 | 3.0 | 5.9×10⁸ | 275 | 0.48×10⁸ |
| 122 000 | 2.2 | 5.6×10⁸ | 275 | 0.33×10⁸ |

The total yield in rhu-a-IFN is 82%, of which 35.6% is referred to fractions with a specific activity comprised between 1x10⁸ U.I./mg and 2.4x10⁸ U.I.

### Example 5

### Purification by cation exchange chromatography of rhu-β-IFN partially purified on CPG^{(R)}-500.

Purpose of the experiment is to verify the effect of the elution rate on the recombinant hu-β-IFN purification. A column of 25.2 cm diameter and 9.6 cm hieght (Pharmacia, model BP 252) was packed with 4780 ml CM Sepharose^{(R)} CL-68. Then 6000 ml of rhu-β-IFN solution partially purified on CPG^{(R)}-500 are fed in the column at a flow rate of 0.1 cm/min; the solution has the following features:
- β-IFN title 4 425 000 U.I./ml
- protein title 0.243 mg/ml
- specific activity 1.80 x 10⁷ U.I./mg

A protein/matrix ratio of 0.31 mg/ml is obtained. At the end of the alimentation and after various washing steps as reported in example 4, the recombinant human β-IFN is eluted using a solution 0.15 M NaCl in 0.1 M phosphate buffer (pH 6.5) at the flow rate of 0.18 cm/min and the fractions containing rhu-β-IFN are collected. The total yield in rhu-β-IFN is 65%, of which only 2.6% shows a specific activity higher than 10.0x10⁷ U.I.

## Claims

1. Process for the chromatographic purification of recombinant human β-interferon characterized in that:
a) an aqueous solution of recombinant human β-interferon in unrefined state is contacted with a siliceous solid matrix equilibrated with deionized water upon which the recombinant human β-interferon is adsorbed, then the recombinant human β-interferon is eluted with an aqueous solution of acetic acid at molar concentration comprised between 2 and 10 mmoles/litre and the eluted fractions containing the recombinant human β-interferon of higher purity level are then collected; and
b) the eluted fractions containing the recombinant human β-interferon purified according to stage a) are loaded with flow rate not exceeding 0.1 cm/min on a cation exchange matrix equilibrated with acetate buffer at pH not lower than 3.0 and the rhu-β-interferon adsorbed upon said matrix is eluted with phosphate buffer of concentration comprised between 0.07 and 0.15 moles/litre, at pH comprised between 6.2 and 6.8 and containing 0.15 to 0.18 moles/litre of NaCl in such a way as to obtain the recombinant human β-interferon as a unique clear peak in determined eluted fractions.

2. Process according to claim 1, wherein the siliceous matrix in stage a) is selected from the group of controlled porosity glass materials (CPG^{(R)}).

3. Process according to claim 2, wherein CPG is CPG^{(R)} 500.

4. Process according to claim 1, wherein the acetic acid concentration in stage a) is comprised between 4 and 7 mmoles/litre.

5. Process according to claim 4, wherein the acetic acid concentration is 5 mmoles/litre.

6. Process according to claim 1, wherein the cation exchange matrixes in stage b) are selected among CM-Sepharose^{(R)}, CM-Sephadex^{(R)}, SP-Sepharose^{(R)}, SP-Sephadex^{(R)} or CM-ACCEL^{(R)}.

7. Process according to claim 6, wherein the cation exchange matrix is CM-Sepharose(R)-CL-6B.

8. Process according to claim 1, wherein the loading and eluting flow rate of recombinant human β-interferon in stage b) are comprised within 0.05 and 0.1 cm/min.

9. Process according to claim 1, wherein the acetate buffer in stage b) has a pH comprised within 3.5 and 4.5.

10. Process according to claim 9, wherein the acetate buffer has pH 4.0.

11. Process according to claim 1, wherein the phosphate buffer is at a concentration comprised between 0.09 and 0.11 moles/litre.

## Patentansprüche

1. Verfahren zur chromatographischen Reinigung von rekombinantem menschlichem β-Interferon dadurch **gekennzeichnet,** daß
a) eine wäßrige-Lösung von rekombinantem menschlichem β-Interferon in ungereinigtem Zustand mit einer siliziumdioxidhaltigen Feststoffmatrix, die mit entionisiertem Wasser äquilibriert ist und an der das rekombinante menschliche β-Interferon adsorbiert wird, in Kontakt gebracht wird; das rekombinante menschliche β-Interferon dann mit einer wäßrigen Essigsäurelösung mit einer molaren Konzentration, die zwischen 2 und 10 mmol/l liegt, eluiert wird, und die eluierten Fraktionen, die das rekombinante menschliche β-Interferon mit höherem Reinheitsgrad enthalten, dann gesammelt werden; und
b) die eluierten Fraktionen, die das entsprechend Schritt a) gereinigte rekombinante menschliche β-Interferon enthalten, mit einer Durchflußgeschwindigkeit, die 0,1 cm/min nicht überschreitet, auf eine Kationenaustauschermatrix aufgebracht werden, welche mit Acetatpuffer mit einem pH von nicht unter 3,0 äquilibriert ist; und das an dieser Matrix adsorbierte rhu-β-Interferon wird mit Phosphatpuffer, der eine Konzentration, die zwischen 0,07 und 0,15 mol/l liegt, einen pH zwischen 6,2 und 6,8 hat und 0,15 bis 0,18 mol/l Nacl enthält, in einer Weise eluiert wird, daß das rekombinante menschliche β-Interferon in bestimmten eluierten Fraktionen als einzelner scharfer Peak erhalten wird.

2. Verfahren nach Anspruch 1, in dem die siliziumdioxidhaltige Matrix im Schritt a) aus der Gruppe von Glasmaterialien kontrollierter Porosität (CPG^{®}) ausgewählt wird.

3. Verfahren nach Anspruch 2, in dem CPG CPG^{®} 500 ist.

4. Verfahren nach Anspruch 1, in dem die Essigsäurekonzentration im Schritt a) zwischen 4 und 7 mmol/l liegt.

5. Verfahren nach Anspruch 4, in dem die Essigsäurekonzentration 5 mmol/l ist.

6. Verfahren nach Anspruch 1, in dem die Kationenaustauschermatrizes im Schritt b) unter CM-Sepharose®, CM-Sephadex®, SP-Sepharose®, SP-Sephadex® oder CM-ACCEL^{®} ausgewählt werden.

7. Verfahren nach Anspruch 1, in dem die Kationenaustauschermatrix CM-Sepharose^{®}-CL-6B ist.

8. Verfahren nach Anspruch 1, in dem die Durchflußgeschwindigkeit beim Aufbringen und Eluieren von rekombinantem menschlichem β-Interferon im Schritt b) zwischen 0,05 und 0,1 cm/min liegt.

9. Verfahren nach Anspruch 1, in dem der Acetatpuffer im Schritt b) einen pH hat, der zwischen 3,5 und 4,5 liegt.

10. Verfahren nach Anspruch 9, in dem der Acetatpuffer einen pH von 4,0 hat.

11. Verfahren nach Anspruch 1, in dem der Phosphatpuffer eine Konzentration hat, die zwischen 0,09 und 0,11 mol/l liegt.

## Revendications

1. Procédé de purification chromatographique d'interféron bêta humain recombinant caractérisé en ce que :
a) une solution aqueuse d'interféron bêta humain recombinant à l'état non raffiné est mise en contact avec une matrice siliceuse solide équilibrée par de l'eau désionisée par suite de quoi l'interféron bêta humain recombinant est adsorbé, puis l'interféron bêta humain recombinant est élué par une solution aqueuse d'acide acétique à une concentration molaire comprise entre 2 et 10 mmoles/litre et les fractions éluées contenant l'interféron bêta humain recombinant de degré de pureté plus élevé sont ensuite recueillies ; et
b) les fractions éluées contenant l'interféron bêta humain recombinant purifié suivant l'étape a) sont chargées avec un débit n'excédant pas 0,1 cm/min sur une matrice échangeuse de cation équilibrée par un tampon acétate à un pH qui n'est pas inférieur à 3,0, et l'interféron bêta humain recombinant adsorbé sur ladite matrice est élué par un tampon phosphate de concentration comprise entre 0,07 et 0,15 moles/litre, à un pH compris entre 6,2 et 6,8 et contenant O,15 à 0,18 moles/litre de Nacl de talle sorte que l'on obtienne l'interféron béta humain recombinant sous forme de pic net unique dans des fractions éluées déterminées.

2. Procédé selon la revendication 1, dans lequel la matrice siliceuse dans l'étape a) est choisie dans le groupe constitué par les matériaux en verre de porosite contrôlee (CPG®).

3. Procédé selon la revendication 2, dans lequel le CPG est CPG^{®} 500.

4. Procédé selon la revendication 1, dans lequel la concentration en acide acétique dans l'étape a) est comprise entre 4 et 7 mmoles/litre.

5. Procédé selon la revendication 4, dans lequel la concentration en acide acétique est de 5 mmoles/litre.

6. Procédé selon la revendication 1, dans lequel les matrices échangeuses de cation dans l'étape b) sont choisies parmi CM-Sepharose®, CM-Sephadex®, SP-Sepharose^{®}, SP-Sephadex®, ou CM-ACCEL®.

7. Procédé selon la revendication 6, dans lequel la matrice échangeuse de cation est CM-Sepharose®-CL-6B.

8. Procédé selon la revendication 1, dans lequel les vitesses de chargement et d'élution de l'interféron bêta humain recombinant dans l'étape b) sont comprises entre 0,05 et 0,1 cm/min.

9. Procédé selon la revendication 1, dans lequel le tampon acétate dans l'étape b) a un pH compris entre 3,5 et 4,5.

10. Procédé selon la revendication 9, dans lequel le tampon acétate a un pH de 4,0.

11. Procédé selon la revendication 1, dans lequel le tampon phosphate est à une concentration comprise entre 0,09 et 0,11 moles/litre.
